# EUROPÄISCHE PATENTSCHRIFT

(11) **EP 2 061 406 B1**
(45) Veröffentlichungstag und Bekanntmachung des Hinweises auf die Patenterteilung: **09.03.2011**
(21) Anmeldenummer: 07801954.4
(22) Anmeldetag: 28.08.2007
(51) Int. Cl.: A61F 5/01

(54) **HANDGELENKORTHESE**
WRIST ORTHOSIS
ORTHÈSE DE POIGNET

(30) Priorität: 04.09.2006 DE 102006041441
(43) Veröffentlichungstag der Anmeldung: 27.05.2009
(73) Patentinhaber: Bauerfeind AG, 07937 Zeulenroda (DE)
(72) Erfinder: BAUERFEIND, Hans, B., 07937 Zeulenroda (DE); REINHARDT, Holger, 47906 Kempen (DE)
(74) Vertreter: Bardehle, Heinz
(86) Internationale Anmeldenummer: PCT/EP2007/007532
(87) Internationale Veröffentlichungsnummer: WO 2008/028588

(56) Entgegenhaltungen:
- EP-A- 1 382 316
- WO-A-02/17827
- DE-U1- 8 806 792
- US-A- 5 759 166
- US-A1- 2004 049 141

## Beschreibung

Die Erfindung bezieht sich auf eine Handgelenkorthese mit einer Manschette, die für den Durchtritt des Daumens geöffnet ist und die mit Stabilisierungsstäben sowie mit mindestens mit einem Spannband zur Fixierung der Orthese am Handgelenk versehen ist, und die zwei nebeneinander liegende Daumenöffnungen zur Aufnahme entweder des linken oder rechten Daumens aufweist, wobei sich ein mittlerer Stabilisierungsstab zwischen den Daumenöffnungen und jeweils an deren jeweiligen Außenseiten ein seitlicher Stabilisierungsstab erstreckt. Eine solche Handgelenkorthese ist aus der US-A-5 759 166 bekannt.

Eine weitere Orthese ist in der PCT-Schrift WO 02/17827 A1 veröffentlicht. Diese bekannte Handgelenkorthese ist jeweils individuell für eine rechte oder linke Hand gestaltet, wozu in der Manschette der Orthese eine entsprechende Öffnung z.B. für den linken Daumen vorgesehen ist. Die bei dieser Handgelenkorthese verwendeten Spannbänder sind an ihren Enden hakenförmig umgebogen, um ein unerwünschtes Herausgleiten der Spannbänder aus an der Manschette angebrachten Ösen zu verhindern. Da diese Gestaltung dieser Orthese, wie gesagt, jeweils entweder für die linke oder die rechte Hand gestaltet ist, die Orthese also nicht von der rechten zur linken Hand bzw. umgekehrt überführt werden kann, bestand ständig das Bedürfnis, eine Handgelenkorthese zur Verfügung zu haben, die sowohl für das linke als auch das rechte Handgelenk direkt geeignet ist. Um diese Aufgabe wenigstens teilweise zu erfüllen, ist die aus der europäischen Patentanmeldung EP 1 382 316 A1 ersichtliche Handgelenkorthese geschaffen worden, bei der für den Daumen in der Manschette eine relativ große Öffnung vorgesehen ist, die es erlaubt, die Orthese sowohl in einer Längsrichtung als auch der umgekehrten Längsrichtung über die Hand und das Handgelenk zu streifen, wobei jeweils nacheinander die rechte und die linke Hand von der Orthese aufgenommen wird, was dadurch ermöglicht ist, dass die Öffnung für den Daumen so groß gestaltet ist, dass die Orthese sowohl auf die rechte als auf die linke Hand aufgezogen werden kann. Allerdings setzt diese Verwendbarkeit für sowohl für die linke als auch die rechte Hand voraus, dass neben der entsprechend groß gestalteten Daumenöffnung in der Orthese Aufnahmen für Stabilisierungsstäbe vorgesehen sind, die bei einem Wechsel z.B. von der linken zur rechten Hand jeweils in entgegengesetzter Richtung in hierfür vorgesehene Taschen eingesetzt werden müssen. Dies bedeutet, dass die Orthese also nicht direkt entweder für die linke oder rechte Hand verwendet werden kann, also jeweils entsprechend umgestaltet werden muss.

Der Erfindung liegt die Aufgabe zugrunde, eine Handgelenkorthese zu schaffen, die ohne Umgestaltung sowohl für die rechte oder linke Hand verwendet werden kann. Hierzu wird die eingangs grundsätzlich erläuterte Orthese erfindungsgemäß derart gestaltet, dass die Daumenöffnungen jeweils zwischen dem mittleren Stabilisierungsstab und dem jeweiligen seitlichen Stabilisierungsstab angeordnet sind und die Stabilisierungsstäbe im Bereich ihrer Erstreckung neben den Daumenöffnungen diesen folgend dreidimensional gewölbt ausgebildet sind.

Aufgrund der Anordnung der zwei getrennt nebeneinander liegenden Daumenöffnungen in der Manschette der Orthese kann in diese von der gleichen Seite her sowohl die linke als auch die rechte Hand problemlos eingeführt werden, wobei jeweils der Daumen in die eine oder andere Daumenöffnung gelangt. Ein Austausch oder irgendeine Veränderung der Stabilisierungsstäbe ist dabei nicht erforderlich, da diese so angeordnet sind, dass sie sowohl in dem Bereich der einen als auch der anderen Daumenöffnung zusammenwirken, so dass bei Verwendung der Handgelenksorthese zunächst für die linke Hand diese dann auch ohne irgendwelche Änderungsmaßnahmen direkt auf die rechte Hand aufgezogen werden kann. Die dabei zur Wirkung kommenden Stabilisierungsstäbe sind in der Manschette so angeordnet, dass sie sich in gleicher Weise sowohl auf die eine als auch auf die andere Daumenöffnung erstrecken, so dass die Orthese für die Verwendung auf einer linken oder rechten Hand ohne weiteres geeignet ist. Dies geschieht also dadurch, dass die Erstreckung der Stabilisierungsstäbe in besonderer Weise derart gestaltet ist, dass sich ein mittlerer Stabilisierungsstab zwischen den Daumenöff nungen erstreckt und an den Daumenöffnungen jeweils gegenüberliegend zu dem mittleren Stabilisierungsstab ein seitlicher Stabilisierungsstab vorgesehen ist, insgesamt also drei Stabilisierungsstäbe, so dass jede Daumenöffnung zwischen dem mittleren Stabilisierungsstab und einem seitlichen Stabilisierungsstab angeordnet ist. Damit lässt sich die Handgelenkorthese ohne irgendwelche Änderung an ihr direkt sowohl auf die linke als auch auf die rechte Hand aufziehen. Um dabei zu verhindern, dass beim Tragen der Handgelenkorthese die Stabilisierungsstäbe einen störenden Druck auf irgendwelche Teile der Hand, insbesondere den Daumen, ausüben können, sind die Stabilisierungsstäbe im Bereich ihrer Erstreckung neben den Daumenöffnungen diesen folgend zweckmäßig dreidimensional gewölbt gestaltet, wodurch die Stabilisierungsstäbe den jeweils in einer Daumenöffnung hindurch tretenden Daumen gewissermaßen umfassen.

Im Sinne einer symmetrischen Ausbildung der Handgelenkorthese und ihrer Spannbänder gestaltet man diese vorteilhaft so, dass die Spannbänder durch am Außenrand der Manschette angebrachte Ösen geführt sind und mittels Klettverschlüssen mit ihrem einen Ende an dem jeweiligen Spannband zwischen den Ösen und ihrem anderen Ende am Rücken dieses Spannbandes, also an den von den Ösen umgelenkten Enden der Spannbänder anlegbar sind und an diesen haften. Auf diese Weise wird erreicht, dass die Enden der Spannbänder sich nur in geringem Maße von der Manschette abheben und somit beim Tragen einer Handgelenkorthese nicht störend in Erscheinung treten.

Man kann die Spannbänder hinsichtlich ihrer Lage an den Ösen auch sichern, was dadurch geschieht, dass die Enden der Spannbänder gegenüber den Ösen derart verbreitert sind, dass die Verbreiterungen zur Verhinderung unbeabsichtigten Gleitens durch die Ösen als Widerhaken wirken. Diese Gestaltung hat gegenüber der Abknickung der Enden der Spannbänder, wie sie in der eingangs genannten Schrift WO 02/17827 A1 erwähnt ist, den Vorteil, dass sie praktisch überhaupt nicht aus dem Umfang der angelegten Handgelenkorthese heraus tritt, da die Verbreiterungen wie ein Widerhaken wirken. Die Spannbänder lassen sich aber trotzdem problemlos in die Ösen einfädeln, da sie in üblicher Weise aus Textilmaterial gefertigt sind und sich leicht seitlich zusammendrücken lassen, um dann durch die Ösen hindurchgeführt zu werden.

Damit die Handgelenkorthese keinen Wärmestau an dem betreffenden Handgelenk hervorrufen kann, gibt man der Manschette und den Stabilisierungsstäben sowie den Spannbändern Durchbrüche, die dafür sorgen, dass Luft ständig an die betreffenden Teile einer Hand vorbringen kann. In den Figuren sind Ausführungsbeispiele der Erfindung dargestellt. Es zeigen:
- Figur 1: ein rechtes Handgelenk mit Blick auf die Handfläche und mit aufgezogener Handgelenkorthese;
- Figuren 2a und b: eine rechte Hand und eine linke Hand mit jeweils aufge- zogener Handgelenkorthese mit Darstellung der Stabilisie- rungsstäbe;
- Figur 3: die drei Stabilisierungsstäbe einer Handgelenkorthese al- lein;
- Figur 4: einen außen neben dem Daumen verlaufenden Stabilisie- rungsstab allein;
- Figur 5: die Handgelenkorthese, abgenommen vom Handgelenk;
- Figur 6: die auf eine rechte Hand aufgezogene Handgelenkorthese mit Blick auf die Spannbänder.

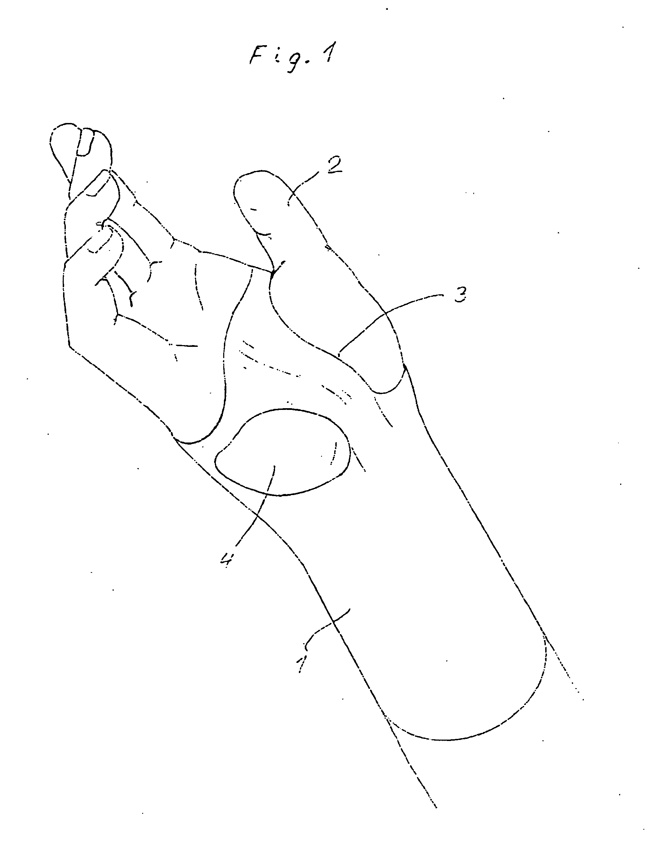

Figur 1 zeigt eine rechte Hand mit der Handgelenkorthese 1, bei der der Daumen 2 durch die eine Daumenöffnung 3 geführt und die andere Daumenöffnung 4 frei ist.

Wenn diese Handgelenkorthese 1 auf eine linke Hand aufgezogen werden würde, dann würde der betreffende Daumen durch die Daumenöffnung 4 hindurch treten.

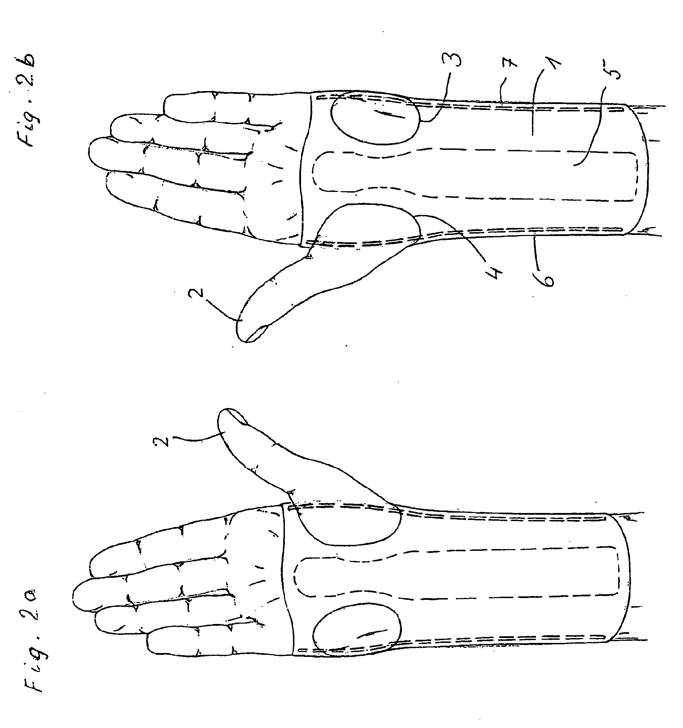

In den Figuren 2a und 2b ist eine rechte Hand und eine linke Hand dargestellt, auf die jeweils die gleiche Handgelenkorthese 1 aufgezogen ist, wobei gemäß Figur 2a der Daumen 2 durch die Daumenöffnung 3 geführt und die Daumenöffnung 4 frei ist.

In Figur 2b ist eine linke Hand mit der Handgelenkorthese 1 versehen, wobei der Daumen 2 durch die Daumenöffnung 4 hindurch tritt, während die Daumenöffnung 3 freigelassen ist.

In den Figuren 2a und 2b sind zusätzlich die Stabilisierungsstäbe 5, 6 und 7 gestrichelt eingezeichnet, da sie in dem Gewebe der Handgelenkorthese 1 in entsprechenden Taschen untergebracht sind, also von außen nicht direkt sichtbar sind. Aufgrund der dargestellten Lage der Stabilisierungsstäbe 5, 6 und 7 ist ersichtlich, dass der Stabilisierungsstab 5 als mittlerer Stabilisierungsstab sich zwischen den Daumenöffnungen 3 und 4 erstreckt, während die seitlichen Stabilisierungsstäbe 6 und 7 jeweils sich außen an der Handgelenkorthese 1 derart erstre cken, dass jede Daumenöffnung 3 und 4 von zwei Stabilisierungsstäben seitlich eingefasst ist, nämlich die Daumenöffnung 3 von den Stabilisierungsstäben 5 und 7 und die Daumenöffnung 4 von den Stabilisierungsstäben 5 und 6. Damit wird im Bereich jeder der beiden Daumenöffnungen 3 und 4 dafür gesorgt, dass neben dem jeweils hindurch tretenden Daumen 2 zwei Stabilisierungsstäbe wirksam werden, nämlich entweder die Stabilisierungsstäbe 5 und 7 oder die Stabilisierungsstäbe 5 und 6, womit innerhalb der Handgelenkorthese 1 eine vollständige Symmetrie herrscht und diese dann also problemlos sowohl auf die rechte Hand (siehe Figur 2a) als auch auf die linke Hand (siehe Figur 2b) aufgezogen werden kann, ohne dass es dabei einer Änderung an der Handgelenkorthese 1 bedarf.

Um auf den jeweils durch eine Daumenöffnung hindurch tretenden Daumen durch die Stabilisierungsstäbe keinen Druck auszuüben, sind diese im Bereich ihrer Erstreckung neben den Daumenöffnungen derart gewölbt ausgebildet, dass sie dreidimensional den Daumen umfassen. Hierzu wird bezüglich des Stabilisierungsstabes 6 auf den Bereich 8 und bezüglich des Stabilisierungsstabes 7 auf den Bereich 9 verwiesen, die bei einem Vergleich zeigen, dass die Bereiche 8 und 9 dreidimensional gewölbt sind, also den Daumen gewissermaßen umschließen, dabei aber in ihrer Querrichtung gewölbt sind, so dass der Daumen gewissermaßen seitlich von ihnen einhüllt wird. Das Gleiche gilt für den Stabilisierungsstab 5, der in dem betreffenden Bereich 10 ebenfalls Wölbungen sowohl in Längsrichtung als in Querrichtung besitzt.

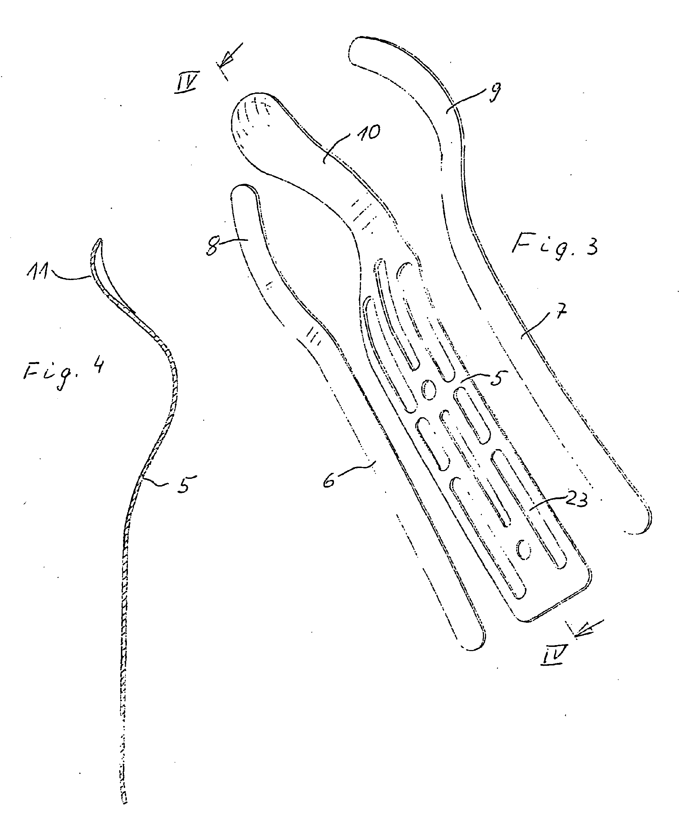

Die Gestaltung des Stabilisierungsstabes 5 ist außerdem als Schnitt längs der Linie IV-IV aus Figur 3 in der Figur 4 dargestellt, aus der hervorgeht, dass dieser Stabilisierungsstab im Bereich 10 sowohl eine längere Rundung als auch zu seinem Ende hin eine Art Löffel 11 bildet, was ebenfalls auf eine dreidimensionale Wölbung hinweist.

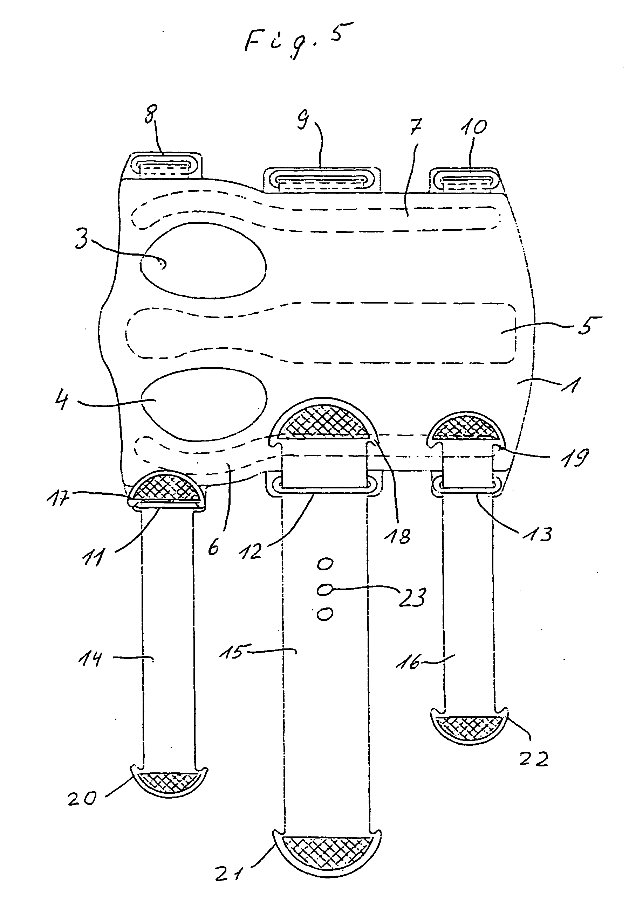

In der Figur 5 ist die Handgelenkorthese 1 im abgenommenen Zustand dargestellt und zwar mit Blick auf den Teil der Orthese, der sich über den Handrücken erstreckt. Die Darstellung gemäß Figur 5 zeigt die Orthese 1 in flach gestreckter Lage mit den beiden Daumenöffnungen 3 und 4 und den mittleren Stabilisierungsstab 5 sowie den seitlichen Stabilisierungsstäben 6 und 7. An der Orthese sind auf ihrer einen Seite die Ösen 8, 9 und 10 angebracht, denen auf der gegenüberliegenden Seite der Orthese die Ösen 11, 12 und 13 gegenüber liegen. Durch die Ösen 11, 12 und 13 sind die Spannbänder 14, 15 und 16 gezogen, die an ihren beiden Enden mit den Verbreiterungen 17, 18 und 19 sowie 20, 21 und 22 versehen sind. Diese Verbreiterungen sind elastisch zusammendrückbar, da die Spannbänder 14, 15 und 16 in bekannter Weise aus einem entsprechend zusammendrückbaren Textilmaterial bestehen. Aufgrund der Verwendung dieser Verbreiterungen wirken diese jeweils wie Widerhaken, so dass sie bei Zug auf die Spannbänder 14, 15 und 16 nicht aus den betreffenden Ösen herausgezogen werden können. Sie können aber aufgrund ihrer Flexibilität ohne weiteres vorher durch die Ösen hindurchgezwängt werden. Um einen durch die angelegte Handgelenkorthese bewirkten Wärmestau in der Hand zu vermeiden, sind in dem manschettenartigen Teil der Orthese sowie den Spannbändern und den Stabilisierungsstäben Durchbrüche vorgesehen, von denen in der Figur 5 bei dem Spannband 15 mehrere Durchbrüche 23 angedeutet sind, die sich in gleicher Weise über die anderen vorstehend genannten Bestandteile der Orthese erstrecken (siehe Figur 3).

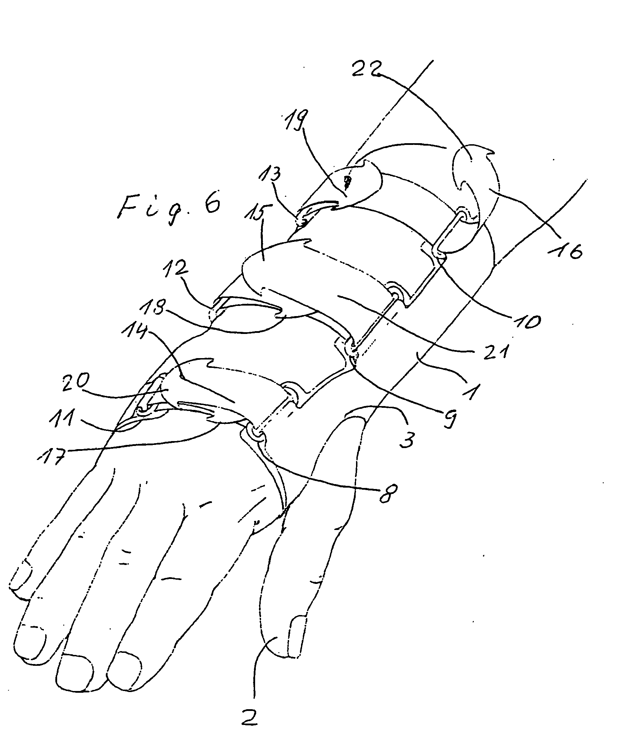

In der Figur 6 ist die an einer rechten Hand angelegte Handgelenkorthese 1 mit Durchtritt des Daumens 2 durch die Daumenöffnung 3 dargestellt, außerdem die Spannbänder 14, 15 und 16, von denen das Spannband 16 in einer Lage vor dem Anheften an der Verbreiterung 19 dargestellt ist. Durch den von der Verbreiterung 22 wegweisenden Pfeil wird angedeutet, dass die Verbreiterung 22 nach Anziehen des Spannbandes 16 an dem Rücken des Spannbandes 16 auf die gegenüberliegende Seite im Bereich der Verbreiterung 19 herangeführt werden kann, wo dann die Verbreiterung 22 mit ihrem Klettverschluss an einen Gegenklettverschluss im Bereich der Verbreiterung 19 angeheftet werden kann. Das Gleiche gilt für das Einziehen und Anlegen der Spannbänder 14 und 15, wie dies aus der Figur 6 deutlich hervorgeht. Jedes der drei Spannbänder 14, 15 und 16 ist mit seinem einen Ende 17, 18 bzw. 19 an dem betreffenden Spannband zwischen den Ösen 11, 12, 13 und 8, 9, 10 (siehe Figur 5) geführt und in diesem Bereich an dem betreffenden Spannband angeheftet, während das jeweils freie Ende der Spannbänder in Form der Verbreiterungen 20, 21 und 22 an den Rücken des betreffenden Spannbandes in Richtung zu der gegenüberliegenden Öse 11, 12, 13 angelegt ist. Damit ist die Handgelenkorthese 1 sicher an dem betreffenden Handgelenk festgelegt.

## Patentansprüche

1. Handgelenkorthese (1) mit einer Manschette, die für den Durchtritt des Daumens geöffnet ist und die mit Stabilisierungsstäben (5, 6, 7) sowie mit mindestens einem Spannband (14, 15, 16) zur Fixierung der Orthese am Handgelenk versehen ist, und die zwei nebeneinander liegende Daumenöffnungen (3, 4) zur Aufnahme entweder des linken oder rechten Daumens (2) aufweist, wobei sich ein mittlerer Stabilisierungsstab (5) zwischen den Daumenöffnungen (3, 4) und jeweils an deren jeweiligen Außenseiten ein seitlicher Stabilisierungsstab (6, 7) erstreckt, **dadurch gekennzeichnet, dass** die Daumenöffnungen jeweils zwischen dem mittleren Stabilisierungsstab (5) und dem jeweiligen seitlichen Stabilisierungsstab (6, 7) angeordnet sind, und die Stabilisierungsstäbe (5, 6, 7) im Bereich ihrer Erstreckung neben den Daumenöffnungen (3,4) diesen folgend dreidimensional gewölbt ausgebildet sind.

2. Handgelenkorthese nach Anspruch 1, **dadurch gekennzeichnet, dass** die Spannbänder (14, 15, 16) durch am Außenrand der Manschette angebrachte Ösen (8, 9, 10; 11, 12, 13) geführt sind und mittels Klettverschlüssen mit ihrem einen Ende (17, 18, 19) an dem jeweiligen Spannband (14, 15, 16) zwischen den Ösen (8, 9, 10; 11, 12,13) und mit ihrem anderen Ende (20,21,22) am Rücken dieses Spannbandes (14,15,16) anlegbar sind.

3. Handgelenkorthese nach Anspruch 1 oder 2, **dadurch gekennzeichnet, dass** die Enden der Spannbänder (17, 18, 19; 20, 21, 22) gegenüber den Ösen (8, 9, 10; 11, 12, 13) derart verbreitert sind, dass die Verbreiterungen zur Verhinderung unbeabsichtigten Gleitens durch die Ösen als Widerhaken wirken.

4. Handgelenkorthese nach einem der Ansprüche 1 bis 3, **dadurch gekennzeichnet, dass** die Manschette, die Spannbänder (17, 18, 19; 20, 21, 22) und die Stabilisierungsstäbe (5, 6, 7) Durchbrüche aufweisen.

## Claims

1. A wrist orthosis including a cuff with an opening for the passage of the thumb therethrough and which is provided with stabilizing rods (5, 6, 7) and with at least one tensioning band (14, 15, 16) for fixing the orthosis to the wrist, and which has two adjacent thumb openings (3, 4) for receiving either the left or the right thumb (2) therein, with a central stabilizing rod (5) extending between said thumb openings (3, 4) and a lateral stabilizing rod (6, 7) each extending on the respective outer sides of said thumb openings (3, 4) **characterized in that** the thumb openings are each disposed between said central stabilizing rod (5) and said respective lateral stabilizing rod (6, 7), said stabilizing rods (5, 6, 7) exhibiting a three-dimensional curvature in the region where they extend next to and along said thumb openings (3, 4).

2. The wrist orthosis of claim 1 **characterized in that** said tensioning bands (14, 15, 16) are guided through eyelets (8, 9, 10; 11, 12, 13) provided on the outer edge of said cuff and include hook-and-loop fasteners allowing one end (17, 18, 19) thereof to be attached to the respective tensioning band (14, 15, 16) between said eyelets (8, 9, 10; 11, 12, 13) and the other end (20, 21, 22) thereof to be attached to the back of said tensioning band (14, 15, 16).

3. The wrist orthosis of claims 1 or 2 **characterized in that** the ends of said tensioning bands (17, 18, 19; 20, 21, 22) are wider than the eyelets (8, 9, 10; 11, 12, 13) and will thus act as "barbed hooks" so as to prevent any accidental slipping of the tensioning bands through said eyelets.

4. The wrist orthosis of one of claims 1 to 3 **characterized in that** said cuff, said tensioning bands (17, 18, 19; 20, 21, 22) and said stabilizing rods (5, 6, 7) are provided with openings.

## Revendications

1. Orthèse pour poignet (1) avec une manchette qui est ouverte pour le passage du pouce et qui est prévue avec des tiges de stabilisation (5, 6, 7) et au moins une sangle (14, 15, 16) pour fixer l'orthèse au poignet et qui présente deux ouvertures pour le pouce côte à côte (3, 4) pour recevoir soit le pouce gauche, soit le pouce droit (2), une tige de stabilisation centrale (5) s'étendant entre les ouvertures pour le pouce (3, 4) et une tige de stabilisation latérale (6, 7) s'étendant entre chaque côté extérieurs respectifs, **caractérisée en ce que** chacune des ouvertures pour le pouce est disposée entre la tige de stabilisation du milieu (5) et chacune des deux tiges de stabilisation latérales (6, 7), et les tiges de stabilisation (5, 6, 7) ont une forme arquée dans la zone se prolongeant près de l'ouverture des pouces (3, 4).

2. Orthèse pour poignet selon la revendication 1, **caractérisée en ce que** les sangles (14, 15, 16) passent par les oeillets (8, 9, 10, 11, 12, 13) adaptés sur les bords extérieurs de la manchette et sont passées entre les oeillets (8, 9, 10, 11, 12, 13) au moyen de velcro situé aux extrémités (17, 18, 19) respectives des sangles (14, 15, 16) et sont appliquées avec leur autre extrémité (20, 21, 22) au dos de cette sangle (14, 15, 16).

3. Orthèse pour poignet selon la revendication 1 ou 2, **caractérisée en ce que** les extrémités des sangles (17, 18, 19, 20, 21, 22) situées face aux oeillets (8, 9, 10, 11, 12, 13) sont disséminées de la sorte que les élargissements pour éviter les glissements non prévus à travers les oeillets jouent le rôle d'ardillons.

4. Orthèse pour poignet selon la revendication 1 à 3, **caractérisée en ce que** la manchette, les sangles (17, 18, 19, 20, 21, 22) et les tiges de stabilisation (5, 6, 7) présentent des ouvertures.
